(19) Europäisches Patentamt

European Patent Office

Office européen des brevets

(11) **EP 2 503 312 B1**

(12) **EUROPEAN PATENT SPECIFICATION**

(45) Date of publication and mention
of the grant of the patent:
**14.11.2018 Bulletin 2018/46**

(51) Int Cl.:
**G01L 9/00** *(2006.01)*    **G01L 11/02** *(2006.01)*
**A61M 5/142** *(2006.01)*    **A61M 5/168** *(2006.01)*

(21) Application number: **11159202.8**

(22) Date of filing: **22.03.2011**

(54) **Medical fluid delivery system with sensor device**

Medizinisches Flüssigkeitsabgabesystem mit einer Sensoranordnung

Système d'administration de liquide médical avec un capteur

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.09.2012 Bulletin 2012/39**

(73) Proprietors:
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**
• **Roche Diabetes Care GmbH**
**68305 Mannheim (DE)**
Designated Contracting States:
**DE**

(72) Inventors:
• **Geipel, Andreas**
**4665, Oftringen (CH)**

• **Grossenbacher, Pascal**
**3006, Bern (CH)**
• **Michel, Philipp**
**3052, Zollikofen (CH)**
• **Haueter, Ulrich**
**3506, Grosshöchstetten (CH)**

(74) Representative: **Rentsch Partner AG**
**Bellerivestrasse 203**
**Postfach**
**8034 Zürich (CH)**

(56) References cited:
**FR-A1- 2 540 987    US-A1- 2009 118 667**

• **KOHL M J ET AL: "A microfluidic experimental platform with internal pressure measurements", SENSORS AND ACTUATORS A, ELSEVIER SEQUOIA S.A., LAUSANNE, CH, vol. 118, no. 2, 28 February 2005 (2005-02-28), pages 212-221, XP025324936, ISSN: 0924-4247, DOI: DOI:10.1016/J.SNA.2004.07.014 [retrieved on 2005-02-28]**

EP 2 503 312 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Field of the Invention

The invention relates to a medical fluid delivery system, in particular for an infusion pump device, like an insulin infusion pump device with a sensor device, to monitor a fluidic pressure in a fluidic system of such system and device. The invention may be used in a method for monitoring the pressure and/or occlusion in a fluidic system.

## State of the art

Medical fluid delivery systems for the administration of e. g. liquid medicament fluids, such as infusion pump devices, are often used with patients who have a continuous, and in the course of the day varying, need of a medicine that can be administered e. g. by subcutaneous infusion. Specific applications are for example certain pain therapies and the treatment of diabetes. In such cases, for example computer controlled automated infusion pump devices are used, which can be carried by the patient on the body, and which contain a certain amount of liquid medicament in a medicine reservoir. The liquid medicament is supplied to the patient's body from the medicine reservoir through a fluidic system to an infusion cannula or an injection needle. Mostly such medical fluid delivery systems comprise a reusable unit including an actuation mechanism, a dosing mechanism, electronics for controlling the mechanisms, etc. and a disposable unit e. g. including a fluid reservoir, which is discarded after emptying the reservoir. Such an Infusion pump device is for example explained in EP 1970677 A1.

The liquid medicament is e. g. obtained by a downstream pump from a flexible container. Flexible containers have the advantage of a smaller volume surplus of the container in relation to its content, which reduces the manufacturing costs and enables design of infusion pump devices of smaller overall dimensions. Thus the device is not evident through clothing and can be carried as comfortably as possible.

In the context of liquid medicament administration via an infusion pump device, sensor devices are used for controlling the dosing, monitoring the correct operation of the system, and for fast detection of faults and hazards, such as occluded infusion lines or cannulae, empty containers, or malfunctioning pump systems. A pressure sensor device may be arranged in a fluid path downstream of a pump device and upstream of an infusion cannula.

Such pressure sensor devices typically comprise a micro-fluidic chamber filled with liquid and fluidly connected to the fluidic system. The chamber is covered by a flexible, resilient membrane, such that a pressure difference between the fluidic pressure inside the sensor chamber and the outside (such as atmospheric) pressure will temporarily deform the membrane. The resulting deflection of the membrane can then be measured by suitable means in order to determine the internal pressure of the fluidic system.

A suitable approach to measure the deformation of the membrane is optical detection of a light beam reflected on the membrane. Such pressure sensor devices are, for example, disclosed in "A microfluidic experimental platform with internal pressure measurements," M. J. Kohl et al., Sensors and Actuators A 118 (2005), pp. 212-221. The pressure sensor device includes a micro-fluidic chamber that is connected to a fluidic system comprising a rigid bottom substrate and a flexible, resilient top cover, for example, a membrane. An optical detection system is arranged to measure a deformation of the cover membrane by determining the interaction of a light beam with the cover membrane. For that purpose a light emitting device, e.g. a laser diode, directs a light beam at a certain angle onto the surface of the cover membrane, where it is reflected. The pressure difference between the inner volume of the microfluidic chamber and the outer environment acts on the cover membrane, and deforms it to a certain extent, depending on the pressure difference. As a result the angle of the reflected light beam changes and the beam is transversely shifted. By monitoring the position of the reflected light beam, the deformation of the cover membrane can be measured, and based on the obtained results a pressure difference value can be determined.

In WO 2007/093064 an insulin pump is shown, which comprises a reusable unit with drive, dosing and controlling systems and a disposable unit with a flow path and a catheter tube. The flow path can be connected to the driving and dosing mechanism, such that a liquid system runs from a liquid reservoir to the catheter tube. A resilient membrane is arranged in the flow path and covers a fluidic chamber. The membrane is impinged directly by the liquid in the flow path, such that it is deflected towards the reusable unit in case of a pressure increase e. g. due to an occlusion of the liquid system. The reusable unit comprises a light emitter in form of a laser diode and a photosensitive x-y sensor, which is connected to the controlling system. The light beam of the laser diode can be directed on a rigid reflector arranged on the membrane. Alternatively the light beam can be directed directly on the membrane surface. In this case a small area on the surface of the membrane is metal-coated, to provide good reflecting properties of the membrane. The incident light beam is focused on an area of the membrane, which is distanced to the center of the membrane. That means in a deflected state of the membrane the reflecting area is inclined and rounded with respect to the non-deflected state. Therefore a reflected light beam changes direction and shape, which can be detected by the x-y sensor, and which is an indication for a pressure change within the liquid system.

An other infusion pump system is disclosed in WO 2008/144693 A1, which comprises an occlusion sensor, that can be used to detect when an occlusion exists in the fluid path between a medicine reservoir and the infusion site. The occlusion sensor includes a flexible membrane, which can be deflected by rising pressure in the fluid path, such that it touches a wall part of an air cavity. The incident measurement light beam is guided through a light transmissive member to the air cavity. In a non-deflected state the incident beam is totally reflected at the air cavity. In a deflected state, when the membrane touches the air cavity, reduced reflection occurs. The reduction of the intensity of the reflected light beam is a measurement for the pressure in the fluid path.

The FR 2540987 A1 discloses an electro-optical pressure sensor that is realized as compact integral unit and is based on focusing a light beam on an inflexion point area of a pressure-deformable membrane and receiving a reflected light beam by a sensor which is arranged such that the reflected light beam is, in the absence of pressure deformation of the membrane, focused on the sensor center.

Since some of the parts of the pressure sensor device are arranged in the disposable unit and other parts are arranged in the reusable unit the orientation of disposable and reusable part towards each other is a critical parameter of reliable measuring results. In the known systems the tolerances between these units must be as small as possible. For being able to monitor the reflected light beam in a pressure sensor according to the prior art, a detector in the optical detection system often is designed to be movable, or a multiplicity of detectors at different positions and at different angles are included in the device. Both of these aspects make such sensor devices expensive and difficult to make. A wide range of directions of reflected light beams due to the large variety or diffuse reflection angles on a membrane also requires a complex sensor array in the pressure sensor device.

### Objects of the Invention

It is an object of the present invention to provide a medical fluid delivery system, particularly in an infusion pump device for liquid medicaments, like insuline, with an improved sensor device which overcomes the drawbacks of prior art devices.

The sensor device may be used in a method for monitoring pressure variations in a medical fluid delivery system, which is easy to install and indicates pressure changes or fluid path occlusion in a simple manner.

### Summary of the invention

A sensor device of a medical fluid delivery system according to the present disclosure comprises a fluidic chamber with a deformable cover closing at least one area of the chamber and an optical detection system comprising at least one light emitter for emitting one or more incident light beams and a sensor unit for monitoring one or more reflected light beams. The fluidic chamber can be realized as part of a fluid path of an infusion pump device or a medical fluid delivery system. It can be formed e. g. as a micro-fluidic chamber, which is in fluidic connection with such a fluid path. A fluidic chamber may e. g. be designed as described in European Application No. 10167585.8 where appropriate for a sensor device according to the present disclosure. The cover can be placed over an opening in the fluidic chamber, such that the cover is in contact with the interior atmosphere of the chamber. The cover may be in direct contact with a fluid delivered through the fluidic chamber. But in general it is possible to locate a transmission volume, like a gas volume, between the fluid and the cover, which transmits pressure changes in the fluid to the cover.

The fluidic chamber is part of a disposable unit of the medical fluid delivery system and the optical detection system is part of a reusable unit of the medical fluid delivery system. The light emitter is for example provided by one or more light emitting diodes (LED) or laser diodes. Advantageously one such diode emitting one light beam of one wavelength is enough to realize the sensor device. The sensor unit can for example be provided as photodiodes.

The cover e. g. is a flexible, resilient membrane cover, which is deformable or deflectable respectively. In a normal state of operation of the sensor device or the medical fluid delivery device, that means a regular pressure atmosphere within the fluidic chamber, the cover in general is essentially not deformed. In case of a medical fluid delivery system for insulin such a regular pressure is e. g. 200 to 2000 mbar relative pressure. This state will be indicated as a non-pressurized state of the fluidic chamber, although a specific normal pressure always exists in the system. The non-pressurized state can be determined by a reference measurement with the optical detection system. Thus the non-pressurized state may serve as a reference state and pressure changes are detected in respect to this reference state. In case the pressure within the fluidic chamber increases or decreases, the cover will be deformed due to the force of the pressure change. This state will be indicated as a pressurized state of the fluidic chamber comprising a deformed cover.

In the pressurized state of the fluidic chamber the deformable cover is deformed such that it forms an inflexion point area within the deformed cover. The inflexion point area is characterized as the area wherein the radius of curvature of the cover changes along a line running within the cover from an edge of the cover connected to the fluidic chamber to the center of the cover. It can also be characterized as the area within the absolute value of the curvature changes sign. In this area the curvature of the cover is negligible in approximation and acts as an even or nearly even reflection area. In fact when the cover is deformed and buckling to one or the other side an inflexion line around the center of the cover is formed. The one or more incident light beams emitted by the light emitter are directed on the cover such that the one or more incident light beams are reflected essentially in the inflexion point area, in particular when the cover is in a deformed state.

In the normal state of the cover, i. e. the non-pressurized state, the incident light beam meets the cover in an area, which corresponds to a potential inflexion point, or in close vicinity of this area. While the fluidic chamber is pressurized to a pressurized state that area is tilted and mostly also offset translative compared to the normal state due to the deformation of the cover. Since the area comprises the inflexion point, it is approximately even and may act as plane reflection area for the incident light beam in the pressurized state. At the same time it is the area with the most angular change compared to the normal state of the cover. That means the one or more light beams are reflected in the area of the cover comprising the largest change of orientation towards the incident light beams, when passing from the non-pressurized state to the pressurized state. But around the inflexion point there is a minimum sphericity negatively affecting the quality of the reflected light beam.

The sensor device according to the disclosure allows a maximum change in reflection of the one or more incident light beams in the pressurized state compared to the non-pressurized state of the cover and therefore ensures accurate observation of pressure abnormalities within the fluidic chamber. The detection at the maximum deflection of the reflected light beams enables a simple and cost-efficient construction of the sensor unit and provides large flexibility in the arrangement of the elements of the sensor device relative to each other.

Good results are achieved by using an incident light beam, which is essentially a parallel light beam. Therefore a laser diode or a light emitting diode comprising an optical element for parallelizing emitted light beams can be used advantageously. In case of a parallel incident light beam and the fact, that the deformed cover is generally planar in the inflexion point area the reflected light beam also is basically parallel and can be focused precisely towards the sensor unit.

Depending on the light emitter used the incident light beam may practically be realized by a bundle of light beams, which covers not only a point on the cover but rather spreads over an area on the cover surface. The diameter of the over all incident light beam should be selected such that an edge of the beam does not extend beyond the center point of the cover on the fluidic chamber. The diameter of the incident light beam may be 1/3 or less of the cover diameter. It is advantageous if it is less than 1/4 and even less than 1/5 of the cover diameter.

In a non-pressurized state of the fluidic chamber the incident one or more light beams are can be directed on the cover at an angle $\alpha$. Alternatively the incident light beams may be focused perpendicular on a non-pressurized cover. An angled direction provides large flexibility in the construction of the single elements of the optical detection system. Furthermore it is advantageous to reflect the one or more incident light beams at an inflexion point area on the cover located closest to the light emitter.

That minimizes the optical path of the incident light beam and maximizes the optical path of the reflected light beam from the inflection point area to the senor unit. Therefore the distance travelled by the reflecting light beam on the sensor unit is maximized and the resolution of the sensor unit is optimized.

Due to the optimized deflection of the incident light beams by the inflexion point area of the fluid chamber cover it is not necessary to provide a sensor unit with a large array of photodiodes. In an advantageous embodiment of the disclosure the sensor unit is provided by a dual-element sensor, which comprises two photo-elements. For example two photodiodes can be arranged directly next to each other in one line. The incident light beam can be focused on a first photodiode, when the fluidic chamber is in a non-pressurized state, and is focused on a second photodiode, when the fluidic chamber is in a pressurized state. That means while a pressure change in the fluidic chamber occurs, the reflected light beam wanders from the first photodiode to the second photodiode. From a particular pressure value on the reflected light beam is fully focused on the second photodiode. The particular pressure value is adapted to correspond to a specific application of the sensor device. For the use of the sensor device as a pressure sensor e. g. in an infusion pump device, the reflected light beam shall change from first to second photodiode within a range of pressure change of 200 mbar to 2000 mbar. Also the material of the cover or the arrangement of the two photodiodes may be adapted to relevant pressure changes and the particular pressure value respectively.

Alternatively the two photodiodes may be arranged separated from each other. In this case at least one optical element may be provided in an optical path from the inflexion point area towards one or both of a first and/or a second photodiode to direct the reflected light beam to the respective photodiode. The optical element or elements are located such that in a non-pressurized state the reflected light beam hits the first photodiode directly or a first optical element directs the beam to the first photodiode. In a pressurized state the reflected light beam is deflected by the inflexion point area of the cover such that it moves away from the first photodiode or the first optical element and instead hits a second optical element, which guides the beam to the second photodiode, or meats the second photodiode directly. A mirror element can for example be used to guide the reflected light beam.

In one embodiment the light emitter of the sensor device according to the disclosure is located on a side of the cover which is curved in a convex manner in an increased pressure state of the fluidic chamber. That means the light emitter directs the one or more incident light beams directly on the cover instead of first passing the interior of the fluidic chamber and the fluid delivered through the chamber. Mostly one side of the cover is in fluid contact and the light emitter is located on the other side. In this arrangement the point of incidence of the incident light beam, which defines the inflexion point area on the cover, is subject to a superposition of two effects when the cover changes from the normal state to the pressurized state and shaping the inflexion point or line, as mentioned above. First the point of incidence is elevated translational by a distance w and second the point of incidence is tilted by an angle ε. That means in the deformed state of the cover the inflexion point area changes its angular and its translational location in respect to the non-deformed state. A distance s travelled on the sensor unit by the reflected light beams, e. g. from the first photodiode to the second phototdiode, can therefore be determined by the superposition in case of small elevation and deflection:

$$s = 2 \cdot w \cdot \cos(\alpha) + d \cdot \tan(2\,\varepsilon),$$

wherein α is defined by the angle of incidence between the incident light beam and the cover and d is the distance from the point of incidence on the cover to sensor unit. The angle α may lie between 30° to 60° and even between 40° to 50°. In this range the distance s acts basically linear to a pressure within the fluidic chamber.

Of course the degree of tilting and elevation depends on the material used for the cover or membrane cover respectively. The cover can be realized as a polymer foil. The polymer foil may be an integral part of the disposable unit of a delivery device or the like. Material used is for example PMMA, PC, PET, PA, PSU, or COC/COP. The thickness of the cover material is for example between 50 μm and 200 μm, advantageously between 80 μm and 150 μm. The cover must reflect a sufficient portion of the incident light beam from the light emitter. Therefore the cover material can be metalized, for example by a layer of aluminium, chrome, silver, gold or the like. Alternatively a metal foil comprising sufficient elasticity may be used.

An infusion pump device may use a medical fluid delivery system, which comprises a sensor device as mentioned above. The sensor device may be an integral part of a dosing unit of the infusion pump device. For example the fluidic chamber of the sensor device is part of a dispensing fluid path connected to a delivery opening of the infusion pump device.

As described the medical fluid delivery system comprises a reusable unit in combination with a disposable unit. The disposable unit comprises for example a fluid reservoir and usually is discarded after use. The reusable unit is designed such that a disposable unit can be connected to it. Also the sensor unit is located in the reusable unit. The optical detection system of the sensor device is part of the reusable unit of the delivery device and the fluidic chamber is part of the disposable unit of the delivery device. The disposable unit comprises an optical element for directing the incident light beam in a predetermined angle α on the inflexion point area of the cover. That means the light emitter can be arranged in any suitable manner relative to the fluidic chamber and the cover respectively, e. g. such that the emitted light is orientated parallel or perpendicular to the cover. The parallel or perpendicular light beam is refracted by the optical element such that light beam is directed to the cover in the desired angle α. Furthermore the disposable unit of the delivery device may comprise an optical element for directing the reflected light from the inflexion point area to the sensor unit as discussed before.

In one embodiment of the medical fluid delivery device according to the disclosure the optical elements for the light path of the incident and reflected light beams may be part of the disposable unit of the medical fluid delivery system. For example the optical elements may be part of a cap or covering of the fluidic chamber, which is attached to a surrounding edge of the cover of the fluidic chamber. The optical elements are transparent for the light of the light emitter. The optical elements are made of amorphous polymer material for example. The fluidic chamber and the optical elements may be made of the same material. With the use of such a disposable unit, the incident light beam of the light emitter can be orientated perpendicular to the cover on the fluidic chamber. The perpendicular light beam is deflected by the first optical element or the covering of the disposable unit such that the incident light beam is directed in angle α on the inflexion point area of the cover. After reflection of the light beam a second optical element may focus the reflected beam onto the sensor unit or one of the photodiodes of the sensor unit respectively.

The use of optical elements facilitates a correct positioning of the disposable and reusable units relative to each other. The optical elements can compensate a mismatch of the units within a tolerance of approximately ± 0.5 mm. Therefore a medical fluid delivery system comprising a sensor device according to the present disclosure enables accurate observation of the functioning of the delivery system.

According to a method for monitoring a pressure change in a fluidic chamber of a medical fluid delivery system according to the present disclosures using a sensor device as described before one or more incident light beams are directed on the inflexion point area of the deformable cover, then one or more light beams reflected from the deformable cover in a non-pressurized state and a pressurized state are detected and the detection data of the non-pressurized state and the pressurized state are compared to extract the pressure change value. Therefore the optical detection system can be connected to an electronic controlling system of the medical fluid delivery system. A threshold pressure level can be defined and deposited in the controlling system, at which the delivery system is shut down or an alarm is given to indicate a critical pressure value or malfunction of the system. In case of an occlusion of a fluidic path of the medical fluid delivery system for example the threshold value may be between 200 mbar and 2000 mbar relative to the atmosphere, mostly it is between 500 mbar and 1200 mbar and it even can be around 1000 mbar.

In a non-pressurized state of the fluidic chamber a reflected light beam reflected from the non-deformed cover of the fluidic chamber can be directed on a first photo-element of the dual-element sensor and in a pressurized state of the fluidic chamber a reflected light beam reflected from the deformed cover can be directed on a second photo-element of the dual-element sensor. The threshold pressure level may e. g. be reached as soon as the reflected beam fully left the first photo-element and fully hits the second photo-element. Alternatively the threshold pressure level may be achieved as soon as a particular value of light intensity is detected on the second photo-element. In a still further alternative, the difference of the light intensity detected by the two photo-elements is evaluated.

The sensor device according offers reliable and simple detection of pressure changes and/or occlusion in a fluid path of the system. It allows a space saving structure of a delivery system and reduces complexity of detection algorithms. Also the sensor device can easily and safely be used in combination with reusable and disposable units of the system and can be produced cost-efficiently.

## BRIEF DESCRIPTION OF THE DRAWINGS

Preferred embodiments of the invention and further related examples will be described in the accompanying drawings, which may explain the principles of the invention but shall not limit the scope of the invention. The drawings illustrate:

Fig.1a: schematic cross-sectional view of an exemplary sensor device in a non-pressurized state,

Fig. 1b: schematic cross-sectional view of the sensor device according to figure 1a in a pressurized state,

Fig. 2a: schematical illustration of a translational offset of the cover in a pressurized state,

Fig. 2b: schematical illustration of an inclination of the cover in a pressurized state,

Fig. 2c: schematical illustration of a superposition of the translational offset and the an inclination of the cover in a pressurized state,

Fig. 3a: diagram of sensor signals of a sensor device using sensor unit comprising a dual-element sensor,

Fig. 3b: diagram of an algorithm to determine a threshold pressure value in accordance to a reference pressure value,

Fig. 4: schematical illustration of a first embodiment of a sensor device according to the disclosure using optical elements to direct incident and reflected light beams,

Fig. 5: schematical illustration of a second embodiment of a sensor device according to the disclosure using optical elements,

Fig. 6: schematical illustration of a third embodiment of a sensor device according to the disclosure using optical elements,

Fig. 7: schematical illustration of a fourth embodiment of a sensor device according to the disclosure using optical elements for splitting reflected light beams,

Fig. 8: schematical illustration of a fifth embodiment of a sensor device according to the disclosure using optical elements for splitting reflected light beams,

Fig. 9: schematic cross-sectional view of a first embodiment of a covering of a disposable unit of a medical fluid delivery system according to the disclosure comprising optical elements to direct incident and reflected light beams, and

Fig. 10: schematic cross-sectional view of a second embodiment of a covering of a disposable unit of a medical fluid delivery system comprising optical elements.

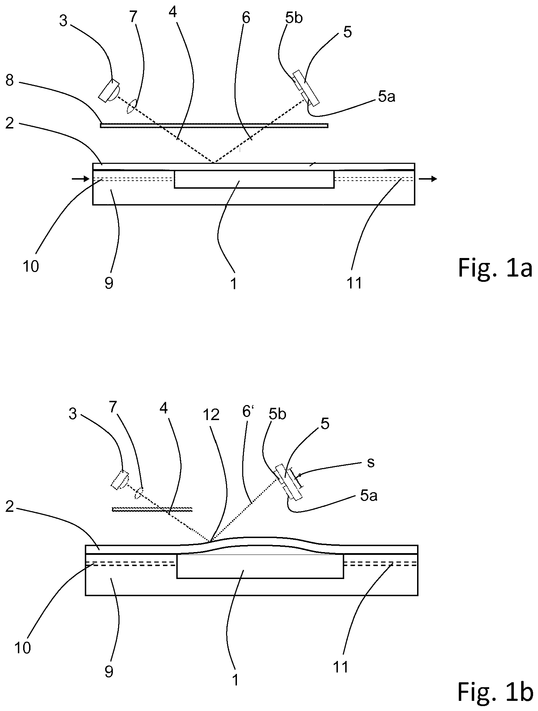

In figures 1 to 10 the basic elements and the function of a sensor device and a method for monitoring pressure changes in an infusion pump device and a medical fluid delivery system are explained with reference to schematic illustrations showing features of the disclosure. The general structure of medical fluid delivery systems or infusion pump devices is know from prior art, e. g. from EP 1970677 A1 or WO 2007/093064 A1. Thus in respect of the general function of a medical fluid delivery system or infusion pump device it is referred to this disclosure.

Figure 1a shows a schematic cross-sectional view of an exemplary sensor device for use in a medical fluid delivery system. The sensor device comprises a fluidic chamber 1 with a deformable cover 2 closing at least one area of the chamber. An optical detection system of the sensor device comprises a light emitter 3, an LED in this case, for emitting incident light beams or a bundle of light beams 4 and a sensor unit 5 for monitoring one or more reflected light beams 6. An optional optical element 7 in form of a collective lens, an aperture, or the like is arranged in the light path of the incident light beam 4 to parallelize the emitted light of the LED 3. The sensor unit 5 in form of a dual-element sensor comprises a first photodiode 5a and a second photodiode 5b, which are arranged directly next to each other in this embodiment. The optical detection system is arranged in a reusable unit of a medical fluid delivery system, wherein the incident light beam and the reflected light beam pass through an optical window 8 of the reusable unit to the exterior of the reusable unit.

The fluidic chamber 1 is part of a fluidic module 9 comprising a cavity, which is covered by the deformable cover 2 on one side. The fluidic chamber 1 has the form of a microfluidic chamber. A fluid inlet 10 connects the fluidic chamber 1 to a fluid reservoir (not shown) and a fluid outlet 11 leads from the fluidic chamber 1 to a patient. The fluid inlet 10, the fluidic chamber 1 and the fluid outlet 11 are part of a fluidic path leading from the fluid reservoir to the patient. The cover may e. g. be glued or welded to the surface of the fluidic module 9. The cover 2 covers an at least partially rounded opening of the fluidic chamber 1. Mostly the opening or cavity is fully circular, which facilitates detecting the cover in a pressurized state and establishing a gentle curvature of the cover. The fluidic module 9 is part of a disposable unit of the medical fluid delivery system, which can be attached to the reusable unit.

In figure 1a the fluidic chamber 1 is in a non-pressurized state, which means the pressure value in the chamber corresponds to normal operation of the medical fluid delivery system as explained before. The deformable cover 2 is not deflected but stretching planar over the fluidic chamber 1. The incident light beam 4 is reflected on the planar surface of the deformable cover 2, such that the reflected light beam 6 is directed on the first photodiode 5a of the sensor unit 5. The photodiode 5a sends a signal to an electronic control system (not shown) of the medical fluid delivery system, which indicates a normal pressure condition within the fluidic path.

In figure 1b the fluidic chamber 1 is shown in a pressurized state. As shown the pressure within the fluidic chamber 1 increased such, that the deformable cover 2 is deformed and forms an inflexion point area 12 within the cover surface covering the fluidic chamber. The deformed cover 2 has a convex shape with respect to the optical detection system and the reusable unit respectively, when the pressure within the fluidic path increases. The incident light beam 4 emitted by the light emitter 3 is directed on the cover 2 such that the incident light beam 4 is reflected essentially in the inflexion point area 12, mostly exactly in the inflexion point. The reflected light beam 6' is deflected due to the change in orientation of the deformable cover 2 in respect to the non-deformed cover by a distance s. The reflected light beam 6' now is directed on the second photodiode 5b of the sensor unit 5. The photodiode 5b sends a signal to the electronic control system, which indicates a pressure change in the fluidic chamber 1 and therefore in the fluidic path.

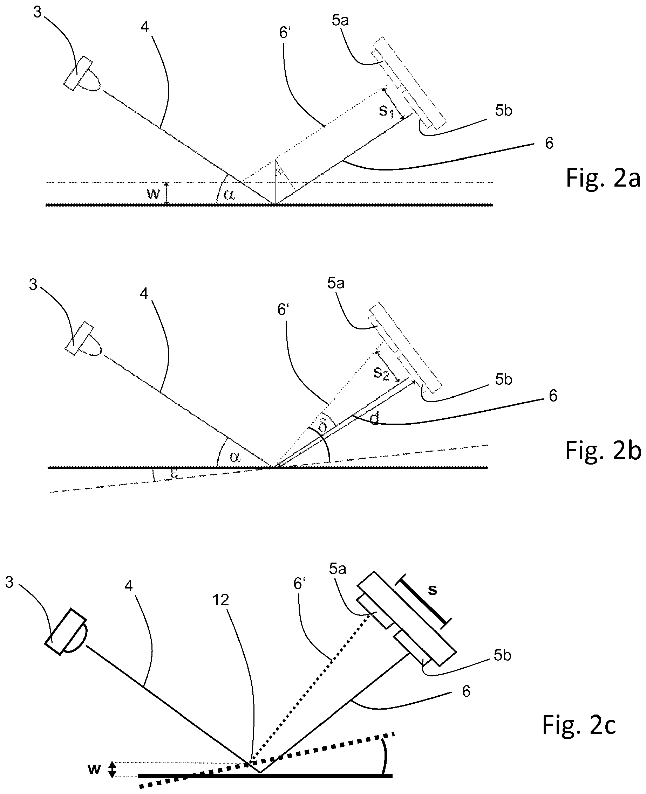

In figures 2a to 2c the change in reflection of the incident light beam 4 due to the deformation of the cover 2 is explained in more detail. In comparison to the normal non-pressurized state in the pressurized state the deformable cover is lifted translational by a height w, as shown in figure 2a. That means the reflected light beam is shifted and offset by a distance $s_1$, which is defined by $s_1 = 2 \cdot w \cdot \cos(\alpha)$, wherein $\alpha$ is the incident angle between the incident light beam and the deformable cover. The reflected light beam 6' of the pressurized state is shifted from the first photodiode 5a in direction of the second photodiode 5b by the distance s1. Also the deformable cover 2 is tilted by an angle $\varepsilon$ relative to the non-pressurized state, which changes the incident angle of the incident light beam, as shown in figure 2b. In the inflexion point area the curvature of the deformed cover changes. The surface of the deformed cover is approximately even and the angle $\varepsilon$ is approximately constant in this area. The direction of the reflected light beam is changed by an angle $\delta$, which is defined by $\delta = 2 \cdot \varepsilon$. That leads to a shift $s_2$ on the sensor unit 5 from the first photodiode 5a in direction of the second photodiode 5b, wherein $s_2 = d \cdot \tan(2\varepsilon)$ and d corresponds to the distance between the reflection point on the deformed cover and the sensor unit 5. As shown in figure 2c, the total shift s of the reflected light beam 6 of the non-pressurized state to a reflected light beam 6' of the pressurized state by a deformation of the cover 2 is given by $s = s_1 + s_2$.

This corresponds to a linear mode of calculation, wherein the total shift s is linear in relation to the pressure in the fluidic chamber 1 approximately. Of course more sophisticated calculation modes may be used. Also the mode of calculation is based on a point-shaped light beam. In practice, in particular when using an LED as light emitter 3, the light beam is rather a bundle of parallel light beams comprising a diameter $D_{lb}$. For using the shown calculation the beam diameter $D_{lb}$ should be selected such, that light at the edge of the bundle does not extend over the center point of the deformable cover 2. The maximum bundle diameter $D_{max}$ should fulfill the following requirement: $D_{lb} \leq 0.58 \sin(\alpha) D_{cov}$, wherein $D_{cov}$ is the diameter of the deformable cover 2. The diameter $D_{cov}$ is measured from a center point of the deformable cover in direction to the inflexion point area chosen for reflecting the incident light beam.

The dual-element sensor 5 generates two separated singular raw signals $S_1$ and $S_2$, one signal $S_1$ of the photodiode 5a and a second signal $S_2$ of the photodiode 5b. In case of a point-shaped light beam, e. g. as emitted by a laser diode, the dual element sensor generates a binary sensor signal S, i. e. one of the raw signals is on and the other one of the raw signal is off. This is because only one of the photodiodes 5a and 5b is energized by the point-shaped light beam at a time. When using a LED as light emitter 3 comprising a bundle of parallel light beams with a diameter $D_{lb}$ a gradual shift from one photodiode 5a to the other photodiode 5b occurs as explained above.

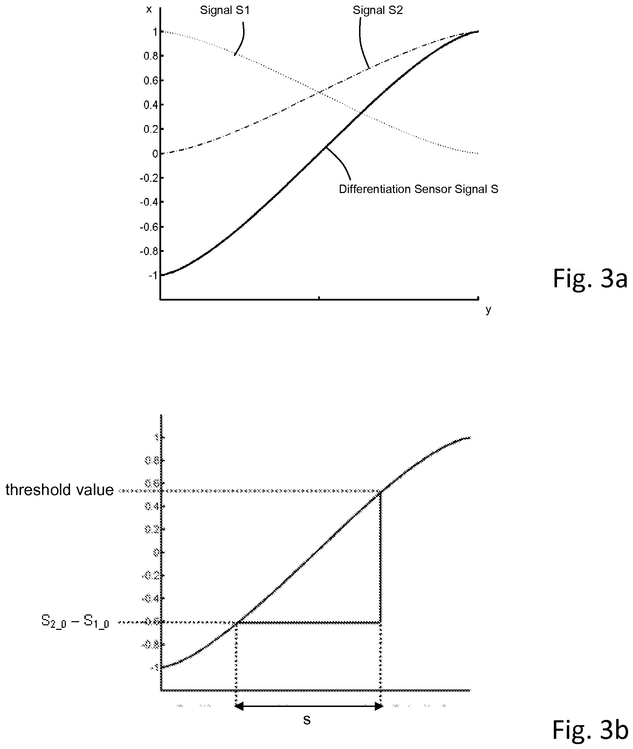

Figure 3a illustrates the characteristics of the raw signals $S_1$ and $S_2$ and a differentiation signal S when a center of the beam bundle is shifted from one center point on photodiode 5a to the adjacent center point on photodiode 5b during deformation of the cover 2, wherein the y-axis indicates the value of the sensor signals and the x-axis indicates the position of the center of the light bundle on the sensor 5. While the beam bundle is centered on the first photodiode 5a raw signal $S_1$ has the value 1 and raw signal $S_2$ has the value 0. During the deformation of the cover 2, the beam bundle wanders to the center of the second photodiode 5b, where raw signal $S_1$ has the value 0 and raw signal $S_2$ has the value 1. By

differentiation $S_2 - S_1$ of the two raw signals the sensor signal S is extracted, which is sufficiently linear in the area of transition between the two photodiodes. Such calculation model allows a quantitative determination of the pressure values in the fluidic chamber.

In practice the exact development of the sensor signal S and the behavior of the reflected light beam depends on the relative positioning between the disposable unit and the reusable unit. The positioning usually lies within a tolerance of $\pm$ 0.2mm and may be different for each disposable unit. Therefore after placing a new disposable unit on the reusable unit the sensor signal S is taken as a reference value, wherein the fluidic pressure on the deformable cover is 0 bar relative to the atmosphere. The reference value allows the integration of the absolute position of the cover before starting the operation of the medical fluid delivery system in the calculation model. Afterwards the usual operation of the system starts for example by priming the fluidic path of the system with fluid.

In case the sensor device according to the present disclosure is used as an occlusion sensor for detecting occlusion of the fluidic path, e. g. for a medical fluid delivery system or an infusion pump device, a characteristic occlusion threshold value is defined, which indicates an occlusion. In a simple algorithm the threshold value may be defined as a fixed value. Alternatively the threshold value of the differentiation sensor signal is provided as a function of the reference value. Thus an influence of disturbing factors, like temperature effects or tolerance variations, can be minimized, which means the threshold value corresponding to occlusion is varied as little as possible. One possibility is to calculate the threshold value of the differentiation sensor signal for each disposable unit at the beginning of the use of the disposable unit by involving the reference value and finally save the threshold value as fixed value. Again the characteristics according to figure 3a are used to describe the sensor signal S. An expected shift s (see above) of the reflected light beam, which corresponds to an occlusion, is known. Therefore based on the reference value, the expected shift s and the characteristics of the sensor signal S the threshold value of the differentiation sensor signal for the occlusion can be determined as shown in figure 3b. The corresponding occlusion requirement is:

$$S_2 - S_1 \geq (S_{2\_0} - S_{1\_0}) + m \cdot s$$

wherein $S_1$ is the raw signal of the first photodiode, $S_2$ is the raw signal of the second photodiode, $S_{1\_0}$ is the reference value of the first photodiode at 0 bar, $S_{2\_0}$ is the reference value of the second photodiode at 0 bar, m is the inclination of the characteristics of the sensor signal S in a linear range, and s is the expected shift of the reflected light beam on the sensor unit.

Another possibility to determine the threshold value of the differentiation sensor signal is to save the reference value of the specific disposable unit and calculate the threshold value at the time of detection.

Non-linear effects in determining an occlusion threshold value can be minimized for example by involving results of experimental investigations. Experiments have shown, that a required correction of a threshold value is bigger, the higher the difference between a reference value and a nominal value corresponding to an ideal situation is. One possible algorithm for defining an occlusion pressure value based on experimental investigations involves only the reference value $S_{1\_0}$ of the first photodiode and is described by a hyperbolic relation as follows:

$$S_2 - S_1 \geq (a \,/\, S_{1\_0}) + c$$

wherein a corresponds to an empirical weighting and c corresponds to an offset value. The weighting a minimizes unwanted non-linear factors in the relevant range of the sensor signal characteristics. The offset value c controls the desired threshold value. The weighting a and the offset value c have to be adapted to a specific design of the sensor device taking into account different basic conditions, e. g. thickness of the cover, diameter of the cover, reflectivity of the cover, etc.. Mostly the threshold value for an occlusion is within the range of 300 mbar and 1500 mbar. Furthermore a temperature factor can be taken into consideration. A temperature sensor can be used to determine temperature changes, which can be located close to the interface between the disposable and reusable unit. Alternatively a forward voltage of the LED may be used as temperature sensitive value.

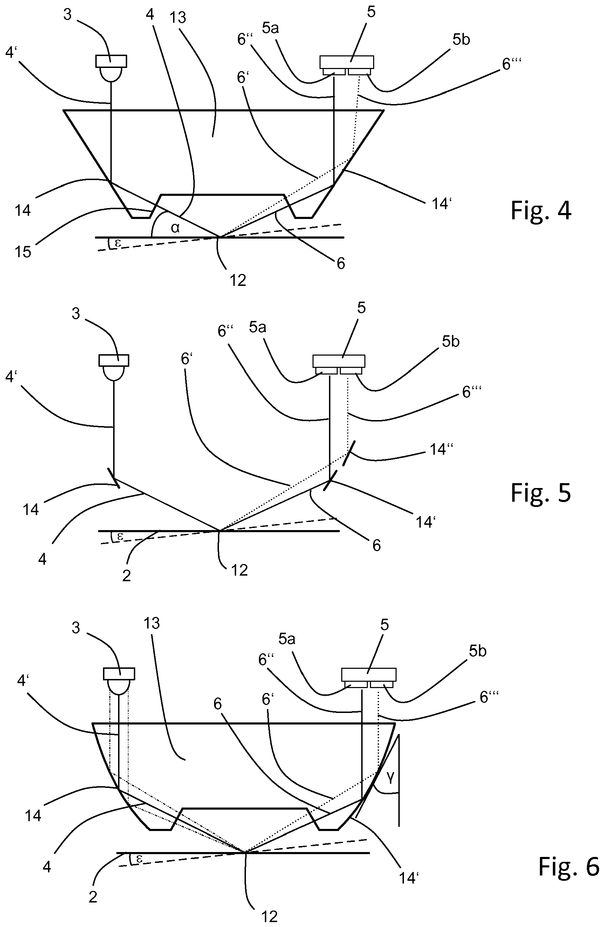

In figures 4 to 8 different embodiments of a sensor device according to the present disclosure are shown, wherein a light emitter 3 does not focus an incident light beam 4 directly on the deformable cover 2 but one or more optical elements are provided, for example as part of a disposable unit of a medical fluid delivery system, which deflect the light beam 4' of the light emitter and directs it as the incident light beam 4 in a predetermined angle on the deformable cover. The incident light beam may reach the deformable cover in an angle between 30° and 60° and mostly in an angle between 40° and 50°. Furthermore an optical element is provided in an optical path from the inflexion point area 12 towards one or both of first and/or second photodiodes. These optical elements deflect the reflected light beam 6 of a non-pressurized state and the reflected light beam 6' of a pressurized state such that they are directed to the sensor unit. The optical elements may be designed as separate elements or realized in an optical unit including several optical elements. The material of the optical elements or unit is mostly transparent, for example an amorphous polymer may be used. Advantageously the optical elements or unit are made of the same material as the fluidic module 9. In general the optical elements or unit are adjusted relative to the deformable cover 2. Such optical elements or units may be used in combination with light emitters 3, which are arranged in a reusable unit of a medical fluid delivery system and emit light beams perpendicular to the deformable cover 2. On the reflection side the reflected light beams may be deflected by the optical elements or unit such, that the reflected light beam perpendicularly enters the reusable unit or the optical window 8 thereof.

Using optical elements allows that the transition of the light beams between a disposable and a reusable unit of a medical fluid delivery system is in general perpendicular, which facilitates positioning of disposable and reusable unit relative to each other, in particular along an orientation perpendicular to the interface surfaces. Furthermore the optoelectronic parts of the sensor device, like the light emitter and the sensor unit, arranged in the reusable unit, can be positioned also in small and narrow constructions of a reusable unit.

In general it is possible to select only a part of the light emitted by the light emitter 3, which corresponds to the direction focusing on the inflexion point area 12 of the cover 2. Thus the lateral position of the disposable unit relative to the reusable unit along the surface of the deformable cover allows more flexibility and tolerances are larger. For example a light-shaping element may be used to confine the emitted light beam.

In figure 4 a first embodiment of a sensor device according to the disclosure is shown using an optical unit 13 to direct the incident and the reflected light beams to and from the inflection point area 12 of the deformable cover 2. The light path of the light beam according to a non-pressurized state is shown as a full line. The light beam 4' emitted by the light emitter 3 enters the optical unit 13 perpendicularly and is reflected at an optical element 14 within the optical unit 13 comprising an angled reflection surface, such that an incident light beam 4 exits the optical unit 13 at an angle $\alpha$ towards the deformable cover 2. A mirror optical element could e. g. be used. An exiting surface 15 is designed such, that it is orientated perpendicular to the incident light beam 4. Alternatively the surface could be designed as a refracting optical element and such contribute to the alignment of the incident light beam. The incident light beam 4 is reflected at the inflexion point area 12. The reflected light beams 6 and 6' are directed back to the optical unit 13, enter the optical unit 13 generally perpendicular and are reflected at a second optical element 14' within the optical element 13. The reflected light beam 6" according to a non-pressurized state is reflected on the optical element 14', exits the optical element 13 and perpendicularly meets the first photodiode 5a of the sensor unit 5. The light path according to a pressurized state is shown as pointed line. The deformation of the deformable cover 2 deflects the reflected light beam 6' as explained above. Therefore the reflected light beam 6' is reflected at the optical element 14' offset from the reflected light beam 6 and is guided towards the photodiode 5b as reflected light beam 6'".

In figure 5 a second embodiment of a sensor device is shown, which is similar to one of figure 4. But the optical elements for guiding the light beams are separated from each other instead of being part of an optical unit1 3. A first optical element 14 is used to reflect the light beam 4' of the light emitter 3. A second optical element 14' is used to reflect the reflected light beam 6 in direction to photodiode 5a. And a third optical element 14" is used to reflect the shifted reflected light beam 6' in direction to photodiode 5b. The single optical elements 14, 14' and 14" can be oriented individually from each other.

In figure 6 a third embodiment of a sensor device is shown, which comprises an optical unit 13 with optical elements 14 and 14' in form of a convex free-forming surface. A bundle of light beams 4' emitted by the light emitter 3 is reflected at the optical element 14 and simultaneously the bundle of light beams 4' is focused on the inflexion point area 12 on the deformable cover 2 by the convex form of the free forming surface. The reflected light beams 6 and 6' are reflected at the convex free-forming surface of the optical element 14'. Due to the convex form the reflected light beam 6" (non-pressurized state) and also the reflected light beam 6'" (pressurized state) run parallel to each other and perpendicular to the sensor unit 5. The angle $\gamma$ at the reflection point of the optical element 14' to focus the reflected light beams 6" and 6'" on the sensor unit 5 is for example defined as $\gamma = 45° - \frac{1}{2}(\alpha + \delta)$.

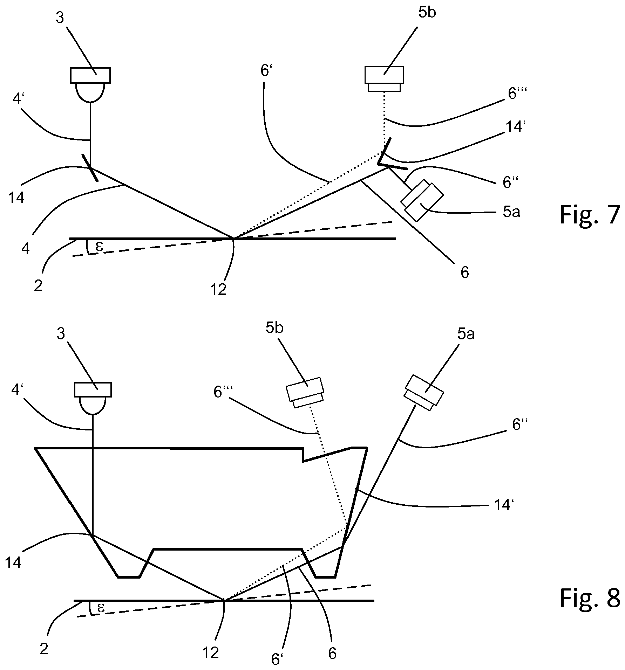

The sensor devices as shown in figures 7 and 8 are advantageously used for the detection of an occlusion of a medical fluid delivery system. The reflected light beam reflected from the deformable cover 2 is deflected at an optical element in largely different directions for a reflected light beam 6 reflected at a non-deformed cover 2 and for a reflected light beam 6' reflected at a deformed cover 2. That means the photo-elements 5a and 5b of the dual-element sensor 5 can be arranged individually and apart from each other.

In figure 7 the incident light beam is directed to the deformable cover 2 as shown in figure 5. The reflected light beams 6 and 6' are directed to an angled optical element 14' comprising two reflecting planes, which are orientated in an angle towards each other. In a non-pressurized state the reflected light beam 6 is directed to a first plane of the optical element 14', which reflects the reflected light beam 6" to the photodiode 5a. In a pressurized state the reflected light beam 6' is directed to a second plane of the optical element 14', which reflects the reflected light beam 6'" to the photodiode 5b. The reflected light beams 6" and 6'" are nearly directed in opposite directions. The angle between first and second plane may be adapted to a desired location of the photodiodes 5a and 5b or according to space requirements in the reusable unit.

In figure 8 the incident light beam is directed to an optical unit 13, which comprises an optical element 14' in form of an optical surface, which acts as a refraction surface for reflected light beams reflected in a non deformed state of the deformable cover 2 and acts as a reflection surface for reflected light beams reflected in a deformed state of the deformable cover 2. In particular the optical surface may be designed such that the reflected light beam is reflected at the optical element 14' as soon as the deformation of the deformable cover 2 corresponds to the occlusion threshold value indicating an occlusion condition of the fluidic module 9. Also the sensor unit 5 may comprise a third photodiode, which is energized by a light beam reflected on the deformable cover 2 in a state in between the non-pressurized state and the pressurized state and is refracted at the optical surface in direction of the third photodiode.

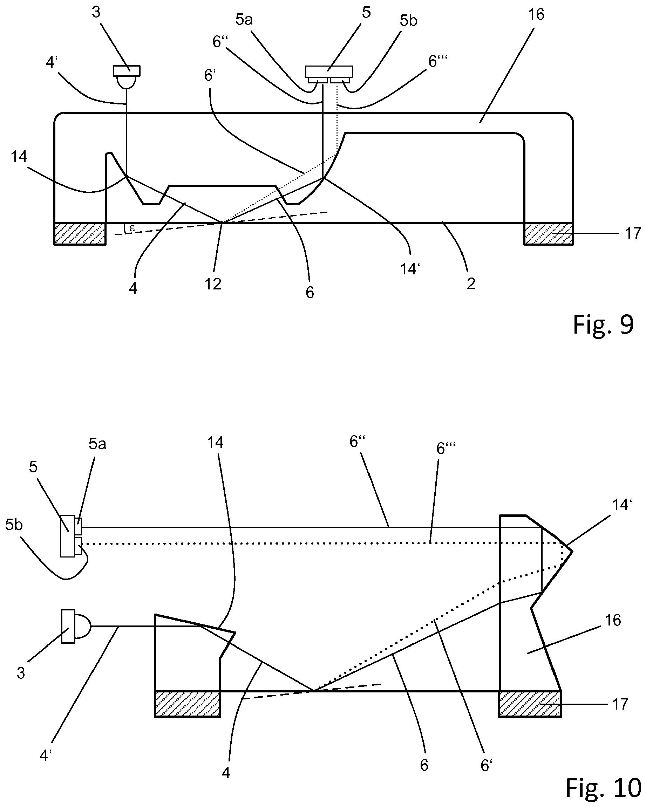

In figures 9 and 10 sensor devices for use in a medical fluid delivery system are shown, wherein optical elements 14 and 14' are part of a cap or covering 16 of the fluidic module 9, which is attached to a surrounding edge 17 of the fluidic module. As shown in figure 9 the covering 16 extends between the optoelectronic parts arranged in the reusable unit of the medical fluid delivery system and the deformable cover 2 and stretches between the edges 17. In direction towards the deformable cover 2 the covering 16 is designed according to an optical unit as shown in figures 4 or 6 respectively and provides optical elements 14 and 14' to direct the incident light beams 4 and 4' and the reflected light beams 6, 6', 6" and 6"'.

In figure 10 an embodiment of the sensor device according to the present disclosure is shown, wherein the light emitter 3 emits a light beam 4' and the sensor unit 5 receives reflected light beams 6" and 6"' parallel to cover 2. That means the optoelectronic parts in the reusable unit can be located on a side of the disposable unit instead of opposite to it. The fluidic module comprises optic elements 14 and 14', wherein a first prism 14 directs the light beam 4' of the light emitter 3 as an incident light beam 4 on the inflexion point area 12 of the deformable cover 2 and a second prism 14' in form of a double prism directs the reflected light beams 6" and 6"' to the sensor unit 5. As explained before the prism 14' is designed such, that the light beam 6 reflected in a non-pressurized state of the deformable cover 2 is directed to the photodiode 5a and the light beam 6' reflected in a pressurized state of the deformable cover 2 is directed to the photodiode 5b.

The present disclosure has been described in respect of several embodiments of a sensor device. Of course the features according to specific described embodiments may be combined to further embodiments by a person skilled in the art although not explained in detail herein.

Reference Numbers

[0059]

| 1 | fluidic chamber |
|---|---|
| 2 | deformable cover |
| 3 | ligth emitter |
| 4, 4' | incident light beam |
| 5 | sensor unit |
| 5a | first photodiode |
| 5b | second photodiode |
| 6, 6', 6", 6"' | reflected light beam |
| 7 | optical parallelizing element |
| 8 | optical window |
| 9 | fluidic module |
| 10 | fluid inlet |
| 11 | fluid outlet |
| 12 | inflexion point area |
| 13 | optical unit |
| 14, 14', 14" | optical element |
| 15 | exiting surface |
| 16 | covering |
| 17 | fluidic chamber edge |

**Claims**

1. Medical fluid delivery system, comprising a sensor device, the sensor device comprising a fluidic chamber (1) with a deformable cover (2) closing at least one area of the chamber and an optical detection system comprising at least one light emitter (3) for emitting one or more incident light beams (4) and a sensor unit (5) for monitoring one or more reflected light beams (6), wherein the fluidic chamber (1) is part of a disposable unit of the medical fluid delivery system and the optical detection system is part of a reusable unit of the medical fluid delivery system, **characterized in that** the sensor device is configured such that one or more incident light beams (6) emitted by the light emitter (3) are directed on the cover (2) such that the one or more incident light beams (4) are reflected essentially in an inflexion point area (12) that is formed within the deformable cover (2) upon deformation of the deformable cover (2) in a pressurized state of the fluidic chamber (1), wherein the disposable unit comprises an optical element (14) configured to direct the one or more incident light beams (4) at a predetermined angle α on the inflexion point area (12) of the cover (2).

2. Medical fluid delivery system according to one of claim 1, **characterized in that** the light emitter (3) of the sensor device is located on a side of the cover (2) which is curved in a convex manner in an increased pressure state of the fluidic chamber (1).

3. Medical fluid delivery system according to one of claims 1 or 2, **characterized in that** one side of the cover (2) is in fluid contact and the light emitter (3) is located on the other side.

4. Medical fluid delivery system according to one the preceding claims, **characterized in that** the incident light beam (4) is a parallel light beam.

5. Medical fluid delivery system according to one the preceding claims, **characterized in that** the light emitter (3) is a laser diode or a light emitting diode comprising an optical element for parallelizing emitted light beams.

6. Medical fluid delivery system according to one the preceding claims, **characterized in that** the sensor unit (5) is provided by a dual-element sensor with two photo-elements (5a, 5b).

7. Medical fluid delivery system according to one the preceding claims, **characterized in that a** diameter of an overall incident light beam ($D_{lb}$) is selected such that an edge of the beam does not extend beyond a center point of the cover (2).

8. Medical fluid delivery system according to one of the preceding claims, **characterized in that** the cover (2) covers an at least partially rounded opening of the fluidic chamber (1).

9. Medical fluid delivery system according to one of the preceding claims, **characterized in that** the one or more incident light beams (4) are reflected at an inflection point area (12) located closest to the light emitter (3).

10. Medical fluid delivery system according to one pf the preceding claims, wherein the disposable unit comprises an optical element (14') for directing the reflected light beam (6) from the inflexion point area (12) to the sensor unit (5).

11. Disposable unit for use with a Medical fluid delivery system according to either of claim 1 to10.


**Patentansprüche**

1. Medizinisches Fluidabgabesystem, umfassend eine Sensorvorrichtung, wobei die Sensorvorrichtung eine Fluidkammer (1) mit einer verformbaren Abdeckung (2), die mindestens einen Bereich der Kammer verschließt, und ein optisches Detektionssystem umfasst, das mindestens einen Lichtemitter (3) zum Ausstrahlen eines oder mehrerer einfallender Lichtstrahlen (4) und eine Sensoreinheit (5) zum Überwachen eines oder mehrerer reflektierter Lichtstrahlen (6) umfasst, wobei die Fluidkammer (1) Teil einer wegwerfbaren Einheit des Abgabesystems für medizinisches Fluid ist und das optische Detektionssystem Teil einer wiederverwendbaren Einheit des Abgabesystems für medizinisches Fluid ist, **da-**

**durch gekennzeichnet, dass**
die Sensorvorrichtung derart konfiguriert ist, dass ein oder mehrere einfallende Lichtstrahlen (6), die von dem Lichtemitter (3) ausgestrahlt werden, derart auf die Abdeckung (2) gerichtet sind, dass die einen oder mehreren einfallenden Lichtstrahlen (4) im Wesentlichen in einem Inflexionspunktbereich (12) reflektiert werden, der innerhalb der verformbaren Abdeckung (2) bei Verformung der verformbaren Abdeckung (2) in einem druckbeaufschlagten Zustand der Fluidkammer (1) ausgebildet ist, wobei die wegwerfbare Einheit ein optisches Element (14) umfasst, das konfiguriert ist, um den einen oder die mehreren einfallenden Lichtstrahlen (4) in einem vorbestimmten Winkel $\alpha$ auf den Inflexionspunktbereich (12) der Abdeckung (2) zu richten.

2. Medizinisches Fluidabgabesystem nach einem von Anspruch 1, **dadurch gekennzeichnet, dass** der Lichtemitter (3) der Sensorvorrichtung auf einer Seite der Abdeckung (2) angeordnet ist, die in einem erhöhten Druckzustand der Fluidkammer (1) in einer konvexen Weise gekrümmt ist.

3. Medizinisches Fluidabgabesystem nach einem der Ansprüche 1 oder 2, **dadurch gekennzeichnet, dass** eine Seite der Abdeckung (2) in Fluidkontakt steht und der Lichtemitter (3) auf der anderen Seite angeordnet ist.

4. Medizinisches Fluidabgabesystem nach einem der vorhergehenden Ansprüche, **dadurch gekennzeichnet, dass** der einfallende Lichtstrahl (4) ein paralleler Lichtstrahl ist.

5. Medizinisches Fluidabgabesystem nach einem der vorhergehenden Ansprüche, **dadurch gekennzeichnet, dass** der Lichtemitter (3) eine Laserdiode oder eine lichtemittierende Diode ist, die ein optisches Element zur Parallelisierung ausgestrahlter Lichtstrahlen umfasst.

6. Medizinisches Fluidabgabesystem nach einem der vorhergehenden Ansprüche, **dadurch gekennzeichnet, dass** die Sensoreinheit (5) von einem Doppelelementsensor mit zwei Fotoelementen (5a, 5b) bereitgestellt wird.

7. Medizinisches Fluidabgabesystem nach einem der vorhergehenden Ansprüche, **dadurch gekennzeichnet, dass** ein Durchmesser eines gesamten einfallenden Lichtstrahls ($D_{lb}$) derart gewählt ist, dass eine Kante des Strahls nicht über einen Mittelpunkt der Abdeckung (2) hinausragt.

8. Medizinisches Fluidabgabesystem nach einem der vorhergehenden Ansprüche, **dadurch gekennzeichnet, dass** die Abdeckung (2) eine mindestens teilweise abgerundete Öffnung der Fluidkammer (1) abdeckt.

9. Medizinisches Fluidabgabesystem nach einem der vorhergehenden Ansprüche, **dadurch gekennzeichnet, dass** der eine oder die mehreren einfallenden Lichtstrahlen (4) an einem Inflexionspunktbereich (12) reflektiert werden, der dem Lichtemitter (3) am nächsten liegt.

10. Medizinisches Fluidabgabesystem nach einem der vorhergehenden Ansprüche, wobei die wegwerfbare Einheit ein optisches Element (14') zum Leiten des reflektierten Lichtstrahls (6) vom Inflexionspunktbereich (12) zur Sensoreinheit (5) umfasst.

11. Wegwerfbare Einheit zur Verwendung mit einem Abgabesystem für medizinisches Fluid nach einem der Ansprüche 1 bis 10.

**Revendications**

1. Système de délivrance de fluide médical, comprenant un dispositif de détection, le dispositif de détection comprenant une chambre à fluide (1) dotée d'un capot déformable (2) fermant au moins une zone de la chambre et un système de détection optique comprenant au moins un émetteur lumineux (3) pour émettre un ou plusieurs faisceaux lumineux incidents (4) et une unité de détection (5) pour contrôler un ou plusieurs faisceaux lumineux réfléchis (6), la chambre à fluide (1) faisant partie d'une unité jetable du système de délivrance de fluide médical et le système de détection optique faisant partie d'une unité réutilisable du système de délivrance de fluide médical, **caractérisé en ce que**
le dispositif de détection est configuré de manière à ce qu'un ou plusieurs faisceaux lumineux incidents (6) émis par les lecteurs lumineux (3) soient dirigés sur le capot (2) de manière à ce que le ou les plusieurs faisceaux lumineux incidents (4) soient réfléchis essentiellement dans une zone de point d'inflexion (12) qui est formée dans le capot déformable (2) en cas de déformation du capot déformable (2) en état pressurisé de la chambre à fluide (1),
l'unité jetable comprenant un élément optique (14) configuré pour diriger un ou plusieurs faisceaux lumineux incidents (4) suivant un angle prédéterminé α sur la zone de point d'inflexion (12) du capot (2).

2. Système de délivrance de fluide médical selon la revendication 1, **caractérisé en ce que** l'émetteur lumineux (3) du dispositif de détection se trouve sur une face du capot (2) qui est courbée de manière convexe dans un état de pression accrue de la chambre à fluide (1) .

3. Système de délivrance de fluide médical selon l'une quelconque des revendications 1 ou 2, **caractérisé en ce qu'**une face du capot (2) est en contact fluidique et que l'émetteur lumineux (3) se trouve sur l'autre face.

4. Système de délivrance de fluide médical selon l'une quelconque des revendications précédentes, **caractérisé en ce que** le faisceau lumineux incident (4) est un faisceau lumineux parallèle.

5. Système de délivrance de fluide médical selon l'une quelconque des revendications précédentes, **caractérisé en ce que** l'émetteur lumineux (3) est une diode laser ou une diode électroluminescente comprenant un élément optique pour paralléliser les faisceaux lumineux émis.

6. Système de délivrance de fluide médical selon l'une quelconque des revendications précédentes, **caractérisé en ce que** l'unité de détection (5) est pourvue d'un capteur à double élément doté de deux photo-éléments de (5a, 5b).

7. Système de délivrance de fluide médical selon l'une quelconque des revendications précédentes, **caractérisé en ce qu'**un diamètre d'un faisceau lumineux incident global ($D_{Ib}$) est sélectionné de manière à ce qu'un bord du faisceau ne s'étende pas au-delà d'un point central du capot (2).

8. Système de délivrance de fluide médical selon l'une quelconque des revendications précédentes, **caractérisé en ce que** le capot (2) couvre une ouverture au moins partiellement arrondie de la chambre à fluide (1).

9. Système de délivrance de fluide médical selon l'une quelconque des revendications précédentes, **caractérisé en ce que** le ou les plusieurs faisceaux lumineux incidents (4) sont réfléchis dans une zone de point d'inflexion (12) située au plus près de l'émetteur lumineux (3).

10. Système de délivrance de fluide médical selon l'une quelconque des revendications précédentes, dans lequel l'unité jetable comprend un élément optique (14') pour diriger le faisceau lumineux réfléchi (6) de la zone de point d'inflexion (12) jusqu'à l'unité de détection (5) .

11. Unité jetable destinée à une utilisation avec un système de délivrance de fluide médical selon l'une quelconque des revendications 1 à 10.

Fig. 1a

Fig. 1b

Fig. 2a

Fig. 2b

Fig. 2c

Fig. 3a

Fig. 3b

Fig. 4

Fig. 5

Fig. 6

Fig. 7

Fig. 8

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1970677 A1
- WO 2007093064 A
- WO 2008144693 A1
- FR 2540987 A1
- EP 10167585 A
- WO 2007093064 A1

### Non-patent literature cited in the description

- **M. J. KOHL et al.** A microfluidic experimental platform with internal pressure measurements. *Sensors and Actuators A,* 2005, vol. 118, 212-221